# EUROPEAN PATENT APPLICATION

(11) **EP 2 343 019 A1**
(43) Date of publication of application: **13.07.2011**
(21) Application number: 11250006.1
(22) Date of filing: 06.01.2011
(51) Int. Cl.: A61B 17/34

(54) **Foam port introduction system including dilator**

(30) Priority: 07.01.2010 US 292974 P; 07.12.2010 US 961560
(71) Applicant: Tyco Healthcare Group LP, New Haven, CT 06511 (US)
(72) Inventor: Farascioni, David, Bethel, CT 06801 (US); Fischvogt, Gregory, Hamden, CT 06514 (US)
(74) Representative: Soames, Candida Jane

(57) **Abstract**

A surgical apparatus (10) for positioning within a tissue tract accessing an underlying body cavity, comprising a compressible seal anchor member (100) including a leading end (104) configured for insertion within the tissue tract and a trailing end (102) configured to remain outside of the tissue tract, the compressible seal anchor member having at least one longitudinal port (108) extending between the leading and trailing ends for substantially sealed reception of an object therein, the compressible seal anchor member including a bore (115) for receipt of a dilator (200).

## Description

### CROSS REFERENCE TO RELATED APPLICATION

The present application claims the benefit of and priority to U.S. Provisional Application Serial No. 61/292,974 filed on January 7, 2010, the entire contents of which are incorporated herein by reference.

### BACKGROUND

### Technical Field

The present disclosure relates generally to a seal for use in a surgical procedure. More particularly, the present disclosure relates to a seal anchor member adapted for insertion into an incision in tissue, and, for the sealed reception of one or more surgical objects such that a substantially fluid-tight seal is formed with both the tissue and the surgical object, or objects.

### Background of Related Art

Minimally invasive procedures are continually increasing in number and variation. Multiple instrument access through a single incision involves performing laparoscopic surgery through a small single incision, often hidden in a site such as the umbilicus. By utilizing a single incision, the potential for wound related complications may be less than it would be if multiple operative sites were to be used. Patients may also benefit from reduced postoperative pain and better cosmetic results.

Some aspects of laparoscopic procedures involving multiple instrument access through a single incision are similar to other laparoscopic procedures. The patient is typically under general anesthesia, insufflated, and laparoscopic visualization is utilized. The maintenance of a substantially fluid-tight seal is desirable to inhibit the escape of insufflation gases and the deflation or collapse of the enlarged surgical site.

Due to the number and variety of laparoscopic techniques, accommodation of multiple surgical instruments within a single incision may necessitate providing a larger sized diameter access port to the internal cavity. It is desirable that an access system be readily inserted and easily advanced into the body while being effectively anchorable and in a sealed relationship with the tissue tract.

### SUMMARY

Disclosed herein is a surgical apparatus for positioning within a tissue tract accessing an underlying body cavity including a seal anchor member. The seal anchor member defines a central longitudinal axis and includes leading and trailing ends, and at least one longitudinal port extending between the leading and trailing ends and being adapted for the substantially sealed reception of an object, e.g., a surgical instrument, therein.

The seal anchor member has a volume and an adjustable compressibility for that volume. The seal anchor member is adapted to transition between a first volume and a second volume. By compressing the seal anchor member to a smaller volume, placement of the seal anchor member within a tissue tract is facilitated. Upon insertion of the seal anchor member within the tissue tract, the seal anchor member is adapted to transition from a compressed insertion volume to an expanded working volume to facilitate a substantially sealed relationship between the seal anchor member and the tissue tract.

In an embodiment, the seal anchor member includes a longitudinally disposed lumen. An empty lumen facilitates the compressibility of the seal anchor member by reducing the amount of compressible material to be compressed and thus reducing the internal biasing force against compression of the seal anchor member, i.e., increasing the compressibility of the seal anchor member. The greater the compressibility of the seal anchor member, the less force is required to place the seal anchor member within a tissue tract.

Once the seal anchor member is placed within the tissue tract, it may be desirable to decrease the compressibility of the seal anchor member. By decreasing the compressibility of the seal anchor member, the pressure exerted between the seal anchor member and the tissue tract is increased and the maintenance of a sealed relationship between the seal anchor member the tissue tract is facilitated.

Compressibility of the seal anchor member is reduced by placing a dilator within the lumen. For a given compressive pressure applied to the seal anchor member, the seal anchor member will compress to a lesser extent with the dilator placed within the lumen as compared to when the lumen is empty. Consequently, placement of the dilator within the lumen of a seal anchor member placed within a tissue tract increases the pressure exerted between the seal anchor member and the tissue surfaces of the tissue tract to facilitate a substantially sealed relationship between the seal anchor member and the tissue surfaces, as compared to when the lumen of the seal anchor member is empty. In some embodiments, placement of the dilator within the lumen may also expand the seal anchor member, e.g., the diameter of the seal anchor member will be increased.

In a further embodiment, the present invention is directed to a surgical apparatus for positioning within a tissue tract accessing an underlying body cavity, comprising: a compressible seal anchor member including a leading end configured for insertion within the tissue tract and a trailing end configured to remain outside of a tissue tracts, the compressible seal anchor member having at least one longitudinal port extending between the leading and trailing ends for substantially sealed reception of an object therein, the compressible seal anchor member including a bore for receipt of a dilator. The compressible seal anchor may have an outer surface between the leading and trailing ends, the outer surface configured to contact the tissue tract. The compressible seal anchor member may have a first volume without the dilator being received in the bore and a second volume with the dilator being received within the bore, the first volume being less than the second volume. The compressible seal anchor member may be more easily inserted into the tissue tract when the dilator is not received within the bore due to its lesser volume. The compressible seal anchor member may provide a first radial pressure without the dilator being received in the bore and a second radial pressure with the dilator being received within the bore, the second radial pressure being greater than the first radial pressure. The compressible seal anchor member may provide for improved sealing with, and retention within, the tissue tract when the dilator is received within the bore due to the greater radial pressure. The object may be a cannula, and the at least one longitudinal port extending between the leading and trailing ends of the compressible seal anchor member may provide for substantially sealed reception of the cannula therein. The compressible seal anchor member may have a first degree of compressibility and the dilator has a second degree of compressibility, e.g., the first degree of compressibility being greater than the second degree of compressibility. The compressible seal anchor member may be formed of a foam material, and the dilator may be substantially rigid.

These and other features of the current disclosure will be explained in greater detail in the following detailed description of the various embodiments.

### BRIEF DESCRIPTION OF THE DRAWINGS

Various embodiments of the present disclosure are described hereinbelow with reference to the drawings, wherein:

Fig. 1 is a perspective view of a seal anchor member in accordance with the present disclosure shown in a first condition relative to a tissue tract;

Fig. 2 is a perspective view of the seal anchor member of Fig. 1 shown in a second condition prior to incision into the tissue tract;

Fig. 3 is a perspective view of a dilator;

Fig. 4 is a perspective view of the seal anchor member of Fig. 1 shown in the first condition with the dilator of Fig. 2 inserted therein; and

Fig. 5 is another embodiment of a seal anchor member in accordance with the present disclosure.

### DETAILED DESCRIPTION OF THE EMBODIMENTS

Particular embodiments of the present disclosure will be described herein with reference to the accompanying drawings. As shown in the drawings and as described throughout the following descriptions, and as is traditional when referring to relative positioning on an object, the term "proximal" will refer to the end of the apparatus that is closest to the clinician during use, and the term "distal" will refer to the end that is farthest from the clinician during use.

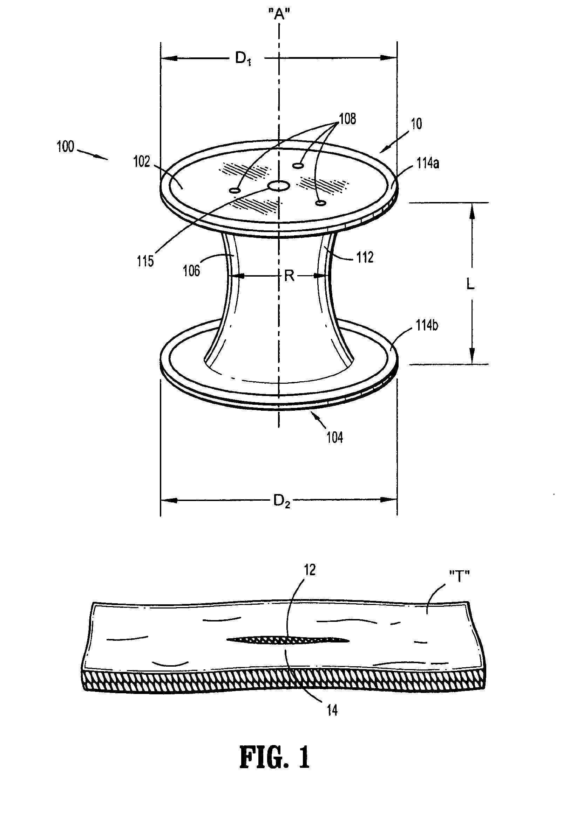

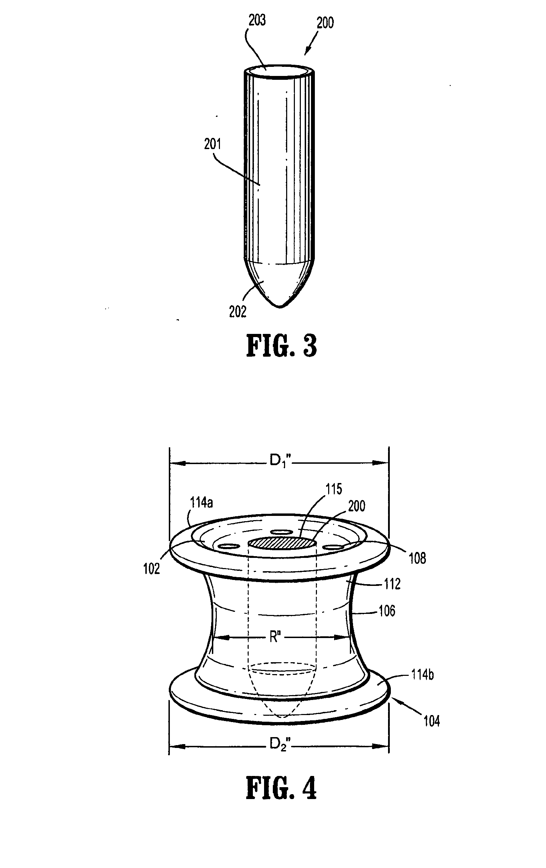

With reference to Figs. 1-4, a surgical apparatus 10 for use in a surgical procedure, e.g., a minimally invasive procedure, is illustrated. The surgical apparatus 10 includes a seal anchor member 100 defining a longitudinal axis "A" and having trailing (or proximal) and leading (or distal) ends 102, 104 and an intermediate portion 106 disposed between the trailing and leading ends 102, 104. The seal anchor member 100 includes one or more ports 108 that extend longitudinally between the trailing end 102 and the leading end 104. Each port 108 is adapted to receive instrumentation (e.g., surgical instruments and/or cannulae) therein in a substantially sealed relation. In addition to ports 108, the seal anchor member 100 also includes a lumen 115 adapted and configured to receive a dilator 200 (Fig. 3).

Proximal end 102 of the seal anchor member 100 defines a first diameter D₁ and distal end 104 defines a second diameter D₂. In an embodiment, the respective first and second diameters D₁ D₂ of the trailing and leading ends 102, 104 are substantially equivalent, as seen in Fig. 1. However, in other embodiments of the present disclosure, the trailing and leading ends 102, 104 may have respective diameters D₁, D₂ that are different. Either or both of trailing and leading ends 102, 104, respectively, define surfaces that are substantially arcuate to assist in the insertion of seal anchor member 100 within a tissue tract 12 defined by tissue surfaces 14 and formed in tissue "T", e.g., an incision, as discussed in further detail below.

Intermediate portion 106 defines a radial dimension "R" and extends longitudinally between proximal and distal ends 102, 104, respectively, to define an axial dimension or length "L". Seal anchor member 100 defines a cross-sectional dimension that varies along length "L", i.e., an hour glass configuration, which facilitates the anchoring of seal anchor member 100 within tissue "T". In alternative embodiments, the radial dimension "R" of the intermediate portion 106 may be substantially equivalent to the respective diameters D₁, D₂ of the proximal and distal ends, 102, 104, respectively. Moreover, in cross-section, intermediate portion 106 may exhibit any suitable configuration, e.g., substantially circular, oval, oblong, or non-circular. Additionally, the intermediate portion 106 may be thinned to ease insertion.

Each port 108 of the seal anchor member 100 is configured to receive a surgical object therein. Prior to the insertion of the surgical object, port 108 is in a first state in which port 108 defines a first or initial dimension. The port 108 may incorporate a slit extending the longitudinal length of the seal anchor member 100. The ports 108 are substantially closed in the absence of a surgical object inserted therein thereby inhibiting the escape of insufflation gas through the port 108. Upon the introduction of a surgical object into the port 108, the port 108 transitions to a second state to substantially approximate the diameter of the surgical object such that a substantially fluid-tight seal is formed therewith, thereby substantially inhibiting the escape of insufflation gas through port 108. In accordance with this embodiment, seal anchor member 100 is formed of a flowable or sufficiently compliable material, such as a foam material, e.g., an open-cell polyurethane foam, a thermoplastic elastomer (TPE) or a gel. The formation of a seal anchor member 100 may involve a process whereby an inert gas, such as carbon dioxide or nitrogen is infused into the material so as to form a foam structure. Seal anchor 100 may also be coated with a lubricious coating, e.g., Parylene N or C, to ease insertion of instruments and/or cannulae therethrough.

As shown in Figs. 1, 2, and 4, the lumen 115 is centered at longitudinal axis "A" of the seal anchor member 100 and is adapted to receive dilator 200 therein. Dilator 200 (Fig. 3) includes a generally cylindrical body section 201, a generally conical tip 202, and a generally flat top surface 203. The shape of the dilator 200 facilitates insertion of the dilator 200 into the lumen 115 of the seal anchor member 200. Upon the introduction of the dilator 200 into lumen 115, the lumen 115 substantially approximates the diameter of the dilator 200 such that a substantially fluid-tight seal is formed therewith, thereby substantially inhibiting the escape of insufflation gas through lumen 115. While the seal anchor member 100 is described herein as having a single longitudinally disposed lumen, it is envisioned that a plurality of lumens may be used and that the lumens may have different orientations within the seal anchor member.

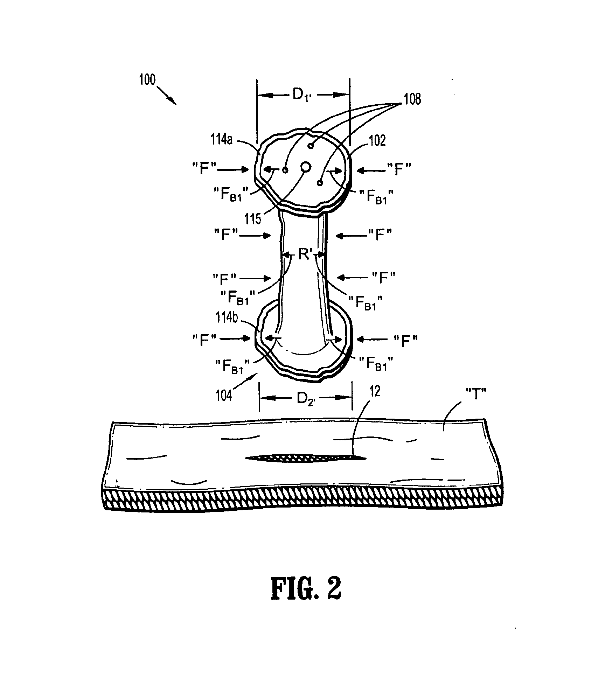

Anchoring of the seal anchor member 100 within the tissue tract 12 is achieved by applying a compressive force "F", as shown in Fig. 2, to reduce the dimensions of the seal anchor member 100, and then inserting the seal anchor member 100 within the tissue tract 12. Application of an external, compressive force "F" to the seal anchor member 100 transitions from an initial condition to a compressed condition. In the initial condition, the seal anchor member 100 is at rest and the trailing end 102 has a radial dimension D₁, the leading end 104 has a radial dimension D₂, and the intermediate portion 106 has a radial dimension R. In the compressed condition, as shown in Fig. 2, the trailing end 102 has a radial dimension D₁', the leading end 104 has a radial dimension D₂', and the intermediate portion 106 has a radial dimension R'.

As depicted in Fig. 2, as seal anchor member 100 is compressed under the influence of external force "F", an internal biasing force "FB₁" within seal anchor member 100 is directed outwardly, opposing force "F". Internal biasing force "F_{BI}" acts to expand seal anchor member 100 and thereby return seal anchor member 100 to its initial, expanded condition. Accordingly, as long as seal anchor member 100 is subject to external force "F" that overcomes the internal biasing force "F_{BI}", seal anchor member 100 remains in the compressed condition. Upon the removal of external "F", however, biasing force "F_{BI}" urges seal anchor member 100 to its initial condition.

Referring again to Fig. 1, one or more positioning members 114a, 114b may be associated with either or both of trailing (or proximal) end 102 and distal (or leading) end 104 of seal anchor member 100. Positioning members 114a, 114b may be composed of any suitable biocompatible material that is at least semi-resilient such that positioning members 114a, 114b may be resiliently deformed and may exhibit any suitable configuration, e.g., substantially annular or oval. Prior to the insertion of seal anchor member 100, positioning members 114a, 114b are deformed in conjunction with the respective proximal and distal ends 102, 104 of seal anchor member 100 to facilitate the advancement thereof through tissue tract 12 (Fig. 2). Subsequent to the insertion of seal anchor member 100 within tissue tract 12, the resilient nature of positioning members 114a, 114b allows positioning members to return to their normal, substantially annular configuration, thereby aiding in the expansion of either or both of the respective proximal and distal ends 102, 104 and facilitating the transition of seal anchor member 100 from its compressed condition to its expanded condition. Positioning members 114a, 114b also may engage the walls defining the body cavity to further facilitate securement of seal anchor member 100 within the body tissue "T". For example, positioning member 114b at leading end 104 may engage the internal peritoneal wall and positioning member 114a, 114b adjacent trailing end 102 may engage the outer epidermal tissue adjacent the incision 12 within tissue "T". In another embodiment of seal anchor member 100, one or more additional positioning members 114a, 114b may be associated with intermediate portion 106.

The use and function of seal anchor member 100 will be discussed during the course of a typical minimally invasive procedure. Initially, the peritoneal cavity is insufflated with a suitable biocompatible gas such as, e.g., carbon dioxide, such that the cavity wall is raised and lifted away from the internal organs and tissue housed therein, providing greater access thereto. The insufflation may be performed with an insufflation needle or similar device, as is conventional in the art. Either prior or subsequent to insufflation, a tissue tract 12 is created in tissue "T", the dimensions of which may be varied dependent upon the nature of the procedure.

In an embodiment, the seal anchor member 100 in its initial state may be configured and dimensioned to facilitate insertion of the seal anchor member 100 into the tissue tract 12. In another embodiment, the seal anchor member 100 in its initial state may have dimensions prohibiting the insertion of the seal anchor member 100 into the tissue tract 12. Insertion of the seal anchor member 100 may be facilitated by transitioning the seal anchor member 100 into the compressed condition by applying a force "F" thereto that is greater than the internal biasing force "F_{BI}", e.g., by squeezing seal anchor member 100. Force "F" acts to reduce the radial dimensions of the proximal and distal ends 102, 104, respectively, to D₁' and D₂' (Fig. 2) including positioning members 114a, 114b (if provided) and to reduce the radial dimension of intermediate portion 106 to R' such that the seal anchor member 100 may be inserted into tissue tract 12. Subsequent to the insertion of the seal anchor member 100, distal end 104, positioning member 114a, 114b (if provided) and at least a section 112 of intermediate portion 106 are disposed beneath the tissue "T". Seal anchor member 100 is caused to transition from the compressed condition to the expanded condition by removing force "F" therefrom. Expansion of the section 112 of the intermediate portion 106 is limited by the tissue surfaces 14 (Fig. 1) defining tissue tract 12, thereby subjecting intermediate portion 106 to an external force "F" that is directed inwardly. As discussed above, this creates an internal biasing force "F_{BI}" that is directed outwardly and exerted upon tissue surfaces 14, thereby creating a substantially fluid-tight seal between the seal anchor member 100 and tissue surfaces 14 and substantially preventing the escape of insufflation gas around seal anchor member 100 and through tissue tract 12.

In the initial condition, the respective radial dimensions D₁, D₂ of the proximal and distal ends 102, 104 are substantially larger than the radial dimension R of the intermediate portion 106 thereby giving seal anchor member 100 an "hour-glass" configuration. Subsequent to insertion, the radial dimension D₂ of distal end 104 and positioning member 114a, 114b is also substantially larger than the dimensions of the tissue tract 12. Consequently, removal of the seal anchor member 100 from tissue tract 12 in the expanded condition is inhibited and thus, seal anchor member 100 will remain anchored within the tissue "T" until it is returned to its compressed condition.

The lumen 115 defines an empty space within the seal anchor member 100. By providing an empty space within the seal anchor member 100, the internal biasing force of the seal anchor member resisting compression is reduced. Accordingly, the force necessary to compress the seal anchor member is less than would be required in the absence of the lumen 115. By reducing the amount of material of the seal anchor member, the force necessary to compress the seal anchor member is reduced and placement of the seal anchor member within the tissue tract is facilitated.

In certain situations, e.g., after placement of the seal anchor member 100 within the tissue tract, it may be desirable to decrease the compressibility of the seal anchor member. Dilator 200 may be inserted within the lumen 115 to resist compression of the seal anchor member 100. Dilator 200 may be placed within lumen 115 subsequent to insertion of the seal anchor member 100 within tissue tract 12 to facilitate a substantially sealed relationship between the tissue surfaces 14 of the tissue tract 12 and the seal anchor member 100. In addition to decreasing the compressibility of the seal anchor member 100, the placement of the dilator 200 within the lumen 115 may increase the radial dimensions of the seal anchor member 100.

Dilator 200 may be formed from the same material as the seal anchor member 100. For example, the dilator 200 may be formed of a flowable or sufficiently compliable material, such as a foam material, e.g., an open-cell polyurethane foam, a thermoplastic elastomer (TPE) or a gel. Alternatively, the dilator 200 may be formed of a different material having the same or different compressibility properties. For example, the dilator 200 may be formed from a rigid material.

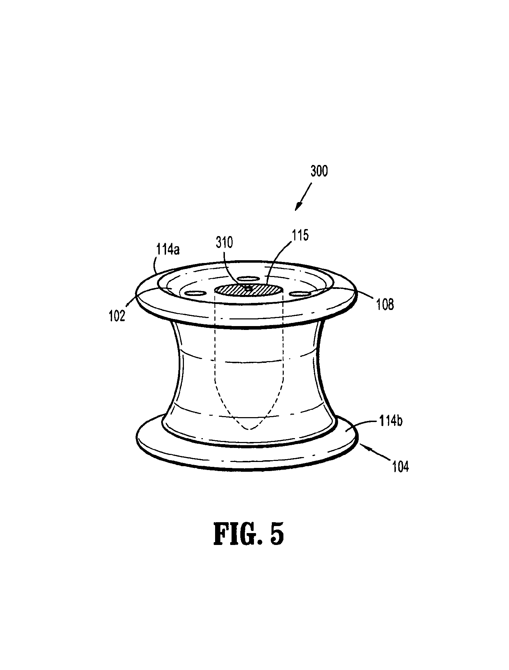

It is envisioned that alternative means may be used to change the compressibility of the seal anchor member. With reference to Fig. 5, an alternative to dilator 200 will now be described. Seal anchor member 300, as shown in Fig. 5, differs from seal anchor member 300 in that lumen 115 is operatively coupled to a valve 310. Valve 310 is adapted to adjust the air pressure within lumen 115 such that the internal biasing force of the seal anchor member 300 may be adjusted. By adjusting the internal biasing force of the seal anchor member 300, the compressibility of the seal anchor member 300 may be adjusted. It may be desirable to have less air within the lumen 115 prior to insertion of the seal anchor member 300 within tissue tract 12. Subsequent to placement of the seal anchor member 300 within the tissue tract 12, the air pressure within the seal anchor member 300 may be increased to facilitate a substantially sealed relationship between the seal anchor member 300 and the tissue tract 12.

Although the illustrative embodiments of the present disclosure have been described herein with reference to the accompanying drawings, the above description, disclosure, and figures should not be construed as limiting, but merely as exemplifications of particular embodiments. It is to be understood, therefore, that the disclosure is not limited to those precise embodiments, and that various other changes and modifications may be effected therein by one skilled in the art without departing from the scope or spirit of the disclosure.

The invention may be described by reference to the following numbered paragraphs:-
1. A surgical apparatus for positioning within a tissue tract accessing an underlying body cavity, comprising:
   a compressible seal anchor member including a leading end configured for insertion within the tissue tract and a trailing end configured to remain outside of a tissue tracts, the compressible seal anchor member having at least one longitudinal port extending between the leading and trailing ends for substantially sealed reception of an object therein, the compressible seal anchor member including a bore for receipt of a dilator.
2. The surgical apparatus of paragraph 1, wherein the compressible seal anchor has an outer surface between the leading and trailing ends, the outer surface configured to contact the tissue tract.
3. The surgical apparatus of paragraph 2, wherein the compressible seal anchor member has a first volume without the dilator being received in the bore and a second volume with the dilator being received within the bore, the first volume being less than the second volume.
4. The surgical apparatus of paragraph 3, wherein the compressible seal anchor member may be more easily inserted into the tissue tract when the dilator is not received within the bore due to its lesser volume.
5. The surgical apparatus of paragraph 2, wherein the compressible seal anchor member provides a first radial pressure without the dilator being received in the bore and a second radial pressure with the dilator being received within the bore, the second radial pressure being greater than the first radial pressure.
6. The surgical apparatus of paragraph 5, wherein the compressible seal anchor member provides for improved sealing with, and retention within, the tissue tract when the dilator is received within the bore due to the greater radial pressure.
7. The surgical apparatus of paragraph 1, wherein the object is a cannula, and the at least one longitudinal port extending between the leading and trailing ends of the compressible seal anchor member provides for substantially sealed reception of the cannula therein.
8. The surgical apparatus of paragraph 1, wherein the compressible seal anchor member has a first degree of compressibility and the dilator has a second degree of compressibility.
9. The surgical apparatus of paragraph 8, wherein the first degree of compressibility is greater than the second degree of compressibility.
10. The surgical apparatus of paragraph 1, wherein the compressible seal anchor member is formed of a foam material.
11. The surgical apparatus of paragraph 1, wherein the dilator is substantially rigid.

## Claims

1. A surgical apparatus for positioning within a tissue tract accessing an underlying body cavity, comprising:
a compressible seal anchor member including a leading end configured for insertion within the tissue tract and a trailing end configured to remain outside of a tissue tracts, the compressible seal anchor member having at least one longitudinal port extending between the leading and trailing ends for substantially sealed reception of an object therein, the compressible seal anchor member including a bore for receipt of a dilator.

2. The surgical apparatus of claim 1, wherein the compressible seal anchor has an outer surface between the leading and trailing ends, the outer surface configured to contact the tissue tract.

3. The surgical apparatus of claim 1 or claim 2, wherein the compressible seal anchor member has a first volume without the dilator being received in the bore and a second volume with the dilator being received within the bore, the first volume being less than the second volume.

4. The surgical apparatus of claim 3, wherein the compressible seal anchor member may be more easily inserted into the tissue tract when the dilator is not received within the bore due to its lesser volume.

5. The surgical apparatus of claim 1 or claim 2, wherein the compressible seal anchor member provides a first radial pressure without the dilator being received in the bore and a second radial pressure with the dilator being received within the bore, the second radial pressure being greater than the first radial pressure.

6. The surgical apparatus of claim 5, wherein the compressible seal anchor member provides for improved sealing with, and retention within, the tissue tract when the dilator is received within the bore due to the greater radial pressure.

7. The surgical apparatus of any preceding claim, wherein the object is a cannula, and the at least one longitudinal port extending between the leading and trailing ends of the compressible seal anchor member provides for substantially sealed reception of the cannula therein.

8. The surgical apparatus of any preceding claim, wherein the compressible seal anchor member has a first degree of compressibility and the dilator has a second degree of compressibility.

9. The surgical apparatus of claim 8, wherein the first degree of compressibility is greater than the second degree of compressibility.

10. The surgical apparatus of any preceding claim, wherein the compressible seal anchor member is formed of a foam material.

11. The surgical apparatus of any preceding claim, wherein the dilator is substantially rigid.
